# EUROPÄISCHE PATENTSCHRIFT

(11) **EP 3 743 496 B1**
(45) Veröffentlichungstag und Bekanntmachung des Hinweises auf die Patenterteilung: **05.01.2022**
(21) Anmeldenummer: 19700872.5
(22) Anmeldetag: 10.01.2019
(51) Int. Cl.: C11D 3/26, C11D 3/28, C11D 3/30, C11D 3/20, C07D 309/38

(54) **WASCH- UND REINIGUNGSMITTEL MIT VERBESSERTER LEISTUNG**
DETERGENTS AND CLEANING AGENTS HAVING IMPROVED PERFORMANCE
PRODUITS DE LAVAGE ET DE NETTOYAGE À EFFICACITÉ AMÉLIORÉE

(30) Priorität: 23.01.2018 DE 102018200960
(43) Veröffentlichungstag der Anmeldung: 02.12.2020
(73) Patentinhaber: Henkel AG & Co. KGaA, 40589 Düsseldorf (DE)
(72) Erfinder: KROPF, Christian, 40724 Hilden (DE); SCHAEFER, Sascha, 40822 Mettmann (DE); UMBREIT, Christian, 41466 Neuss (DE); SCHULZ, Alexander, 45128 Essen (DE); STROTZ, Michael, 50969 Köln (DE); MÜLLER, Thomas J.J., 40591 Düsseldorf (DE); MAYER, Bernhard, 41468 Neuss (DE); DENISSEN, Melanie, 51379 Leverkusen (DE)
(86) Internationale Anmeldenummer: PCT/EP2019/050523
(87) Internationale Veröffentlichungsnummer: WO 2019/145158

(56) Entgegenhaltungen:
- DE-A1-102013 226 003
- US-A- 2 907 772
- CRISTIANO BENELLI ET AL: "Di-maltol-polyamine ligands to form heterotrinuclear metal complexes: solid state, aqueous solution and magnetic characterization", DALTON TRANSACTIONS, Bd. 42, Nr. 16, 1. Januar 2013 (2013-01-01), Seite 5848, XP055361559, ISSN: 1477-9226, DOI: 10.1039/c3dt32130d

## Beschreibung

Die vorliegende Erfindung betrifft die Verwendung bestimmter Bis-4H-Pyranonylmethane in Wasch- und Reinigungsmitteln zur Verbesserung der Wasch- oder Reinigungsleistung.

Während die Formulierung pulverförmiger, Bleichmittel enthaltender Wasch- und Reinigungsmittel heute keinerlei Probleme mehr bereitet, stellt die Formulierung stabiler flüssiger, Bleichmittel enthaltender Wasch- und Reinigungsmittel nach wie vor ein Problem dar. Aufgrund des üblicherweise Fehlens des Bleichmittels in flüssigen Wasch- und Reinigungsmitteln werden solche Anschmutzungen, die normalerweise insbesondere aufgrund der enthaltenen Bleichmittel entfernt werden, entsprechend häufig nur in unzureichender Weise entfernt. Ein ähnliches Problem besteht auch für Bleichmittel-freie Color-Waschmittel, bei denen das Bleichmittel weggelassen wird, um die Farbstoffe im Textil zu schonen und deren Ausbleichen zu verhindern. Bei fehlendem Bleichmittel kommt erschwerend hinzu, dass anstatt der Entfernung der sogenannten bleichbaren Anschmutzungen, die normalerweise durch das Bleichmittel auf Persauerstoffbasis wenigstens anteilsweise entfernt werden, aufgrund des Waschvorgangs häufig im Gegenteil sogar eine Intensivierung und/oder Verschlechterung der Entfernbarkeit der Anschmutzung herbeigeführt wird, was nicht zuletzt auf initiierte chemische Reaktionen zurückzuführen sein dürfte, die beispielsweise in der Polymerisierung bestimmter in den Anschmutzungen enthaltener Farbstoffe bestehen können.

Derartige Probleme treten insbesondere bei Anschmutzungen auf, die polymerisierbare Substanzen enthalten. Bei den polymerisierbaren Substanzen handelt es sich vor allem um polyphenolische Farbstoffe, vorzugsweise um Flavonoide, insbesondere aus der Klasse der Anthocyanidine oder Anthocyane. Die Anschmutzungen können insbesondere durch Lebensmittelprodukte oder Getränke verursacht worden sein, die entsprechende Farbstoffe enthalten. Bei den Anschmutzungen kann es sich insbesondere um Flecken von Früchten oder Gemüse oder auch Rotweinflecken handeln, die insbesondere polyphenolische Farbstoffe, vor allem solche aus der Klasse der Anthocyanidine oder Anthocyane, enthalten.

Aus der internationalen Patentanmeldung WO 2011/023716 A1 ist beispielsweise die Verwendung von Gallussäureestern wie Propylgallat in Wasch- und Reinigungsmitteln zur verbesserten Entfernung von Anschmutzungen, die polymerisierbare Substanzen enthalten, bekannt.

Die Verwendung von am N-Atom gegebenenfalls mit organischen Gruppen, wie der Methyl-, Ethyl-, Propyl-, Phenyl-, Naphthyl- oder Carboxyethylgruppe, substituierten 4-Pyridinonen zur Entfernung von Anschmutzungen von Textilien ist aus der internationalen Patentanmeldung WO 2007/042140 A2 bekannt.

Das Dimere der Kojisäure und seine Komplexbildungseigenschaft für Eisen wurde von R.C. Fox und P.D.Taylor in Bioorganic & Medicinal Chem. Lett. 8 (1998), 443-446 beschrieben. Aus der internationalen Patentanmeldung WO 2015/091124 A1 ist die Verbesserung der Wasch- oder Reinigungsleistung von Wasch- und Reinigungsmitteln in Bezug auf bleichbare Anschmutzungen durch Bis-Pyranonylmethane bekannt.

Überraschenderweise wurde nun gefunden, dass durch den Einsatz bestimmter Bis-4H-Pyranonyl-verbindungen, bei denen die 4H-Pyranonyleinheiten durch Heteroalkylgruppen verbunden sind, die Wasch oder Reinigungsleistung von Wasch- oder Reinigungsmitteln insbesondere in Bezug auf bleichbare Anschmutzungen deutlich verbessert werden kann.

Ein erster Gegenstand der vorliegenden Erfindung ist daher die Verwendung von Verbindungen der allgemeinen Formel (I), 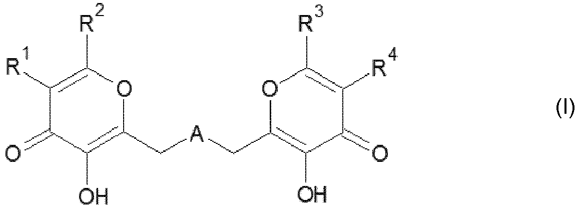 in der A für -Q-(CH₂)ₙ-Q, -(CH₂)ₘ-Q-(CH₂)ₚ- oder 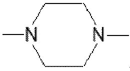 steht, Q für O, N(R⁵) oder N⁺H(R⁵) X⁻ steht, R¹, R², R³, R⁴ und R⁵ unabhängig voneinander für Wasserstoff oder eine geradkettige oder verzweigte Alkylgruppe mit 1 bis 4 C-Atomen stehen, X⁻ für ein ladungsausgleichendes Anion steht, n für eine Zahl von 1 bis 10 steht, und m und p unabhängig voneinander für Zahlen von 0 bis 7 stehen,
in Wasch- oder Reinigungsmitteln zur Verbesserung der Wasch- oder Reinigungsleistung gegenüber bleichbaren Anschmutzungen.

Die bleichbaren Anschmutzungen enthalten üblicherweise polymerisierbare Substanzen, insbesondere polymerisierbare Farbstoffe, wobei es sich bei den polymerisierbaren Farbstoffen vorzugsweise um polyphenolische Farbstoffe, insbesondere um Flavonoide, vor allem um Anthocyanidine oder Anthocyane oder Oligomere dieser Verbindungen handelt. Neben der Entfernung von Anschmutzungen in den Farben grün, gelb, rot oder blau kommt auch die von Anschmutzungen in Zwischenfarben, insbesondere violett, lila, braun, purpurfarben oder rosa, und auch von Anschmutzungen in Betracht, die eine grüne, gelbe, rote, violette, lilafarbene, braune, purpurfarbene, rosafarbene oder blaue Tönung aufweisen, ohne im Wesentlichen selbst komplett aus dieser Farbe zu bestehen. Die genannten Farben können insbesondere auch jeweils hell oder dunkel sein. Es handelt sich hierbei vorzugsweise um Anschmutzungen, insbesondere um Flecken von Gras, Früchten oder Gemüse, insbesondere auch um Anschmutzungen durch Lebensmittelprodukte, wie beispielsweise Gewürze, Saucen, Chutneys, Currys, Pürees und Marmeladen, oder Getränke, wie beispielsweise Kaffee, Tee, Weine und Säfte, die entsprechende grüne, gelbe, rote, violette, lilafarbene, braune, purpurfarbene, rosafarbene und/oder blaue Farbstoffe enthalten.

Die erfindungsgemäß zu entfernenden Anschmutzungen können insbesondere verursacht sein durch Kirsche, Morelle, Traube, Apfel, Granatapfel, Aronia, Pflaume, Sanddorn, Açai, Kiwi, Mango, Gras, oder Beeren, vor allem durch rote oder schwarze Johannisbeeren, Holunderbeeren, Brombeeren, Himbeeren, Blaubeeren, Preiselbeeren, Kronsbeeren, Erdbeeren oder Heidelbeeren, durch Kaffee, Tee, Rotkohl, Blutorange, Aubergine, Tomate, Karotte, Rote Beete, Spinat, Paprika, rotfleischige oder blaufleischige Kartoffel, oder rote Zwiebel.

Verbindungen der allgemeinen Formel (I) können aus Maltol durch Halogenierung der Methylgruppe und Umsetzung mit Bisaminen oder durch Oxidation der Methylgruppe zum Aldehyd, anschließende Reduktion zum Alkohol, gegebenenfalls Aktivierung des Alkohols, zum Beispiel als Methansulfonsäurederivat, und anschließende Umsetzung mit Dihalogeniden hergestellt werden. Zu Verbindungen mit m > 0 und/oder p > 0 gelangt man zum Beispiel durch Kombination dieser Methoden ausgehend von Maltol oder Homologen des Maltols, die in der 2-Position Alkylgruppen mit m+1 oder p+1 C-Atomen tragen.

In den Verbindungen der allgemeinen Formel (I) sind vorzugsweise R¹ und R³ und/oder R² und R⁴ gleich. Zu den bevorzugten Verbindungen der allgemeinen Formel (I) gehören auch solche, in denen R¹, R², R³ und R⁴ Wasserstoff sind, und/oder R⁵ eine Methylgruppe ist. Bevorzugt ist n eine Zahl von 1 bis 6, insbesondere 1 bis 3. Das Anion X⁻, falls vorhanden, wird vorzugsweise ausgewählt aus Lactat, Citrat, Tartrat oder Succinat, Perchlorat, Tetrafluoroborat, Hexafluorophosphat, Alkylsulfonat, Alkylsulfat, Hydrogensulfat, Sulfat, Dihydrogenphosphat, Hydrogenphosphat, Phosphat, Isocyanat, Rhodanid, Nitrat, Fluorid, Chlorid, Bromid, Hydrogencarbonat oder Carbonat, wobei bei mehrwertigen Anionen der Ladungsausgleich durch entsprechend mehrere kationische erfindungsgemäße Substanzen oder gegebenenfalls durch die Anwesenheit zusätzlicher Kationen wie Natrium-, Kalium oder Ammoniumionen gewährleistet wird.

Die erfindungsgemäße Verwendung der Verbindung der allgemeinen Formel (I) erfolgt in Wasch- oder Reinigungsmitteln vorzugsweise dadurch, dass man sie in einer Menge von 0,001 Gew.-% bis 20 Gew.-%, insbesondere in einer Menge von 0,01 Gew.-% bis 10 Gew.-%, einsetzt, wobei sich hier und im Folgenden die Angaben von "Gew.-%" jeweils auf das Gewicht des gesamten Wasch- oder Reinigungsmittels beziehen. Ein weiterer Gegenstand der Erfindung ist daher ein Wasch- oder Reinigungsmittel, enthaltend eine Verbindung der oben definierten allgemeinen Formel (I) in einer Menge von vorzugsweise 0,001 Gew.-% bis 20 Gew.-%, insbesondere 0,01 Gew.-% bis 10 Gew.-%, wobei die vor- oder nachstehend beschriebenen bevorzugten Ausführungsformen der erfindungsgemäßen Verwendung sinngemäß auch für diesen Gegenstand der Erfindung gelten. Ein solches Mittel wird in üblichen maschinell oder manuell auszuführenden Wasch- oder Reinigungsverfahren eingesetzt, bei denen verschmutze Wäsche oder eine verschmutzte harte Oberfläche einer das Mittel enthaltenden wässrigen Flotte ausgesetzt wird mit dem Ziel, die Anschmutzung von der textilen oder harten Oberfläche zu entfernen.

Das Wasch- oder Reinigungsmittel kann in jeder nach dem Stand der Technik etablierten und/oder jeder zweckmäßigen Darreichungsform vorliegen. Dazu zählen beispielsweise feste, pulverförmige, flüssige, gelförmige oder pastöse Darreichungsformen, gegebenenfalls auch aus mehreren Phasen bestehend; ferner gehören beispielsweise dazu: Extrudate, Granulate, Tabletten oder Pouches, sowohl in Großgebinden als auch portionsweise abgepackt.

Die erfindungsgemäße Verwendung erfolgt hierbei in einer bevorzugten Ausführungsform in einem Wasch- und Reinigungsmittel, das keine oxidativen Bleichmittel enthält. Hierunter ist zu verstehen, dass das Mittel keine oxidativen Bleichmittel im engeren Sinne, zu denen Hypochlorite, Wasserstoffperoxid oder Wasserstoffperoxid liefernde Substanzen und Peroxosäuren zu rechnen sind, enthält; vorzugsweise weist es auch keine Bleichaktivatoren und/oder Bleichkatalysatoren auf.

Bei dem Waschmittel handelt es sich in einer besonders bevorzugten Ausführungsform um ein flüssiges Textilwaschmittel.

Bei dem Waschmittel handelt es sich in einer weiteren besonders bevorzugten Ausführungsform um ein pulverförmiges oder flüssiges Color-Waschmittel, also ein Textilwaschmittel für gefärbte Textilien.

Die Wasch- oder Reinigungsmittel können neben dem erfindungswesentlichen Wirkstoff übliche sonstige Bestandteile von Wasch- oder Reinigungsmitteln, insbesondere Textilwaschmitteln, enthalten, insbesondere ausgewählt aus der Gruppe der Gerüststoffe und Tenside sowie vorzugsweise der Polymere, Enzyme, Desintegrationshilfsmittel, Duftstoffe und Parfümträger.

Zu den Gerüststoffe zählen insbesondere die Zeolithe, Silikate, Carbonate, organische Cobuilder und - sofern keine ökologischen Vorurteile gegen ihren Einsatz bestehen - auch die Phosphate. Der feinkristalline, synthetische und gebundenes Wasser enthaltende Zeolith ist vorzugsweise Zeolith A und/oder Zeolith P. Als Zeolith P kommt beispielsweise Zeolith MAP^{®} (Handelsprodukt der Firma Crosfield) in Frage. Geeignet sind jedoch auch Zeolith X sowie Mischungen aus Zeolith A, X und/oder P. Kommerziell erhältlich und im Rahmen der vorliegenden Erfindung einsetzbar ist beispielsweise auch ein Co-Kristallisat aus Zeolith X und Zeolith A (ca. 80 Gew.-% Zeolith X), das durch die Formel

n Na₂O . (1-n) K₂O · Al₂O₃ · (2 - 2,5) SiO₂ · (3,5 - 5,5) H₂O

beschrieben werden kann. Der Zeolith kann dabei sowohl als Gerüststoff in einem granularen Compound eingesetzt, als auch zu einer Art "Abpuderung" einer granularen Mischung, vorzugsweise einer zu verpressenden Mischung verwendet werden, wobei üblicherweise beide Wege zur Inkorporation des Zeoliths in das Vorgemisch genutzt werden. Zeolithe können eine mittlere Teilchengröße von weniger als 10 µm (Volumenverteilung; Meßmethode: Coulter Counter) aufweisen und enthalten vorzugsweise 18 Gew.-% bis 22 Gew.-%, insbesondere 20 Gew.-% bis 22 Gew.-% an gebundenem Wasser.

Es können auch kristalline schichtförmige Silikate der allgemeinen Formel NaMSiₓO₂ₓ₊₁ · y H₂O eingesetzt werden, worin M Natrium oder Wasserstoff darstellt, x eine Zahl von 1,9 bis 22, vorzugsweise von 1,9 bis 4 ist, wobei besonders bevorzugte Werte für x 2, 3 oder 4 sind, und y für eine Zahl von 0 bis 33, vorzugsweise von 0 bis 20 steht. Die kristallinen schichtförmigen Silikate der Formel NaMSiₓO₂ₓ₊₁ · y H₂O werden beispielsweise von der Firma Clariant GmbH (Deutschland) unter dem Handelsnamen Na-SKS vertrieben. Beispiele für diese Silikate sind Na-SKS-1 (Na₂Si₂₂O₄₅ · × H₂O, Kenyait), Na-SKS-2 (Na₂Si₁₄O₂₉ · × H₂O, Magadiit), Na-SKS-3 (Na₂Si₈O₁₇ · × H₂O) oder Na-SKS-4 (Na₂Si₄O₉ · × H₂O, Makatit).

Bevorzugt sind kristalline Schichtsilikate der Formel NaMSiₓO₂ₓ₊₁ · y H₂O, in denen x für 2 steht. Insbesondere sind sowohl ß- als auch δ-Natriumdisilikate Na₂Si₂O₅ · y H₂O sowie weiterhin vor allem Na-SKS-5 (α-Na₂Si₂O₅), Na-SKS-7 (ß-Na₂Si₂O₅, Natrosilit), Na-SKS-9 (NaHSi₂O₅ · H₂O), Na-SKS-10 (NaHSi₂O₅ · 3 H₂O, Kanemit), Na-SKS-11 (t-Na₂Si₂O₅) und Na-SKS-13 (NaHSi₂O₅), insbesondere aber Na-SKS-6 (δ-Na₂Si₂O₅) bevorzugt. Wasch- oder Reinigungsmittel enthalten vorzugsweise einen Gewichtsanteil des kristallinen schichtförmigen Silikats der Formel NaMSiₓO₂ₓ₊₁ · y H₂O von 0,1 Gew.-% bis 20 Gew.-%, bevorzugt von 0,2 Gew.-% bis 15 Gew.-% und insbesondere von 0,4 Gew.-% bis 10 Gew.-%.

Einsetzbar sind auch amorphe Natriumsilikate mit einem Modul Na₂O : SiO₂ von 1:2 bis 1:3,3, vorzugsweise von 1:2 bis 1:2,8 und insbesondere von 1:2 bis 1:2,6, welche vorzugsweise löseverzögert sind und Sekundärwascheigenschaften aufweisen. Die Löseverzögerung gegenüber herkömmlichen amorphen Natriumsilikaten kann dabei auf verschiedene Weise, beispielsweise durch Oberflächenbehandlung, Compoundierung, Kompaktierung/Verdichtung oder durch Übertrocknung hervorgerufen worden sein. Unter dem Begriff "amorph" wird verstanden, dass die Silikate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen, hervorrufen.

Alternativ oder in Kombination mit den vorgenannten amorphen Natriumsilikaten können röntgenamorphe Silikate eingesetzt werden, deren Silikatpartikel bei Elektronenbeugungsexperimenten verwaschene oder sogar scharfe Beugungsmaxima liefern. Dies ist so zu interpretieren, dass die Produkte mikrokristalline Bereiche der Größe zehn bis einige Hundert nm aufweisen, wobei Werte bis max. 50 nm und insbesondere bis max. 20 nm bevorzugt sind. Derartige röntgenamorphe Silikate weisen ebenfalls eine Löseverzögerung gegenüber den herkömmlichen Wassergläsern auf. Insbesondere bevorzugt sind verdichtete/kompaktierte amorphe Silikate, compoundierte amorphe Silikate und übertrocknete röntgenamorphe Silikate.

Diese(s) Silikat(e), vorzugsweise Alkalisilikate, besonders bevorzugt kristalline oder amorphe Alkalidisilikate, sind, wenn vorhanden, in Wasch- oder Reinigungsmitteln in Mengen von 3 Gew.-% bis 60 Gew.-%, vorzugsweise von 8 Gew.-% bis 50 Gew.-% und insbesondere von 20 Gew.-% bis 40 Gew.-% enthalten.

Auch ein Einsatz der allgemein bekannten Phosphate als Buildersubstanzen ist möglich, sofern ein derartiger Einsatz nicht aus ökologischen Gründen vermieden werden soll. Unter der Vielzahl der kommerziell erhältlichen Phosphate haben die Alkalimetallphosphate unter besonderer Bevorzugung von Pentanatrium- und Pentakaliumtriphosphat (Natrium- und Kaliumtripolyphosphat) in der Wasch- und Reinigungsmittel-Industrie die größte Bedeutung.

Alkalimetallphosphate ist dabei die summarische Bezeichnung für die Alkalimetall- (insbesondere Natrium- und Kalium-) Salze der verschiedenen Phosphorsäuren, bei denen man Metaphosphorsäuren (HPO₃)ₙ und Orthophosphorsäure H₃PO₄ neben höhermolekularen Vertretern unterscheiden kann. Die Phosphate vereinen dabei mehrere Vorteile in sich: Sie wirken als Alkaliträger, verhindern Kalkbeläge auf Maschinenteilen bzw. Kalkinkrustationen in Geweben und tragen überdies zur Reinigungsleistung bei. Technisch besonders wichtige Phosphate sind das Pentanatriumtriphosphat, Na₅P₃O₁₀ (Natriumtripolyphosphat) sowie das entsprechende Kaliumsalz Pentakaliumtriphosphat, K₅P₃O₁₀ (Kaliumtripolyphosphat). Bevorzugt eingesetzt werden weiterhin die Natriumkaliumtripolyphosphate. Werden Phosphate in Wasch- oder Reinigungsmitteln eingesetzt, so enthalten bevorzugte Mittel diese(s) Phosphat(e), vorzugsweise Alkalimetallphosphat(e), besonders bevorzugt Pentanatrium- oder Pentakaliumtriphosphat (Natrium- bzw. Kaliumtripolyphosphat), in Mengen von 5 Gew.-% bis 80 Gew.-%, vorzugsweise von 15 Gew.-% bis 75 Gew.-% und insbesondere von 20 Gew.-% bis 70 Gew.-%.

Weiter einsetzbar sind Alkaliträger. Als Alkaliträger gelten beispielsweise Alkalimetallhydroxide, Alkalimetallcarbonate, Alkalimetallhydrogencarbonate, Alkalimetallsesquicarbonate, die genannten Alkalisilikate, Alkalimetasilikate, und Mischungen der vorgenannten Stoffe, wobei bevorzugt die Alkalicarbonate, insbesondere Natriumcarbonat, Natriumhydrogencarbonat oder Natriumsesquicarbonat eingesetzt werden. Besonders bevorzugt kann ein Buildersystem enthaltend eine Mischung aus Tripolyphosphat und Natriumcarbonat sein. Aufgrund ihrer im Vergleich mit anderen Buildersubstanzen geringen chemischen Kompatibilität mit den übrigen Inhaltsstoffen von Wasch- oder Reinigungsmitteln werden die Alkalimetallhydroxide üblicherweise nur in geringen Mengen, vorzugsweise in Mengen unterhalb 10 Gew.-%, bevorzugt unterhalb 6 Gew.-%, besonders bevorzugt unterhalb 4 Gew.-% und insbesondere unterhalb 2 Gew.-%, eingesetzt. Besonders bevorzugt werden Mittel, welche bezogen auf ihr Gesamtgewicht weniger als 0,5 Gew.-% und insbesondere keine Alkalimetallhydroxide enthalten. Bevorzugt ist der Einsatz von Carbonat(en) und/oder Hydrogencarbonat(en), vorzugsweise Alkalicarbonat(en), besonders bevorzugt Natriumcarbonat, in Mengen von 2 Gew.-% bis 50 Gew.-%, vorzugsweise von 5 Gew.-% bis 40 Gew.-% und insbesondere von 7,5 Gew.-% bis 30 Gew.-%.

Als organische Builder sind insbesondere Polycarboxylate/Polycarbonsäuren, polymere Polycarboxylate, Asparaginsäure, Polyacetale, Dextrine sowie Phosphonate zu nennen. Brauchbar sind beispielsweise die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Die freien Säuren besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln. Insbesondere sind hierbei Citronensäure, Bernsteinsäure, Glutarsäure, Adipinsäure, Gluconsäure und beliebige Mischungen aus diesen zu nennen. Als Gerüststoffe sind weiter polymere Polycarboxylate geeignet, dies sind beispielsweise die Alkalimetallsalze der Polyacrylsäure oder der Polymethacrylsäure, beispielsweise solche mit einer relativen Molekülmasse von 500 g/mol bis 70000 g/mol. Geeignet sind insbesondere Polyacrylate, die bevorzugt eine Molekülmasse von 2000 g/mol bis 20000 g/mol aufweisen. Aufgrund ihrer überlegenen Löslichkeit können aus dieser Gruppe wiederum die kurzkettigen Polyacrylate, die Molmassen von 2000 g/mol bis 10000 g/mol, und besonders bevorzugt von 3000 g/mol bis 5000 g/mol, aufweisen, bevorzugt sein. Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 Gew.-% bis 90 Gew.-% Acrylsäure und 50 Gew.-% bis 10 Gew.-% Maleinsäure enthalten. Ihre relative Molekülmasse, bezogen auf freie Säuren, beträgt im allgemeinen 2000 g/mol bis 70000 g/mol, vorzugsweise 20000 g/mol bis 50000 g/mol und insbesondere 30000 g/mol bis 40000 g/mol. Zur Verbesserung der Wasserlöslichkeit können die Polymere auch Allylsulfonsäuren, wie beispielsweise Allyloxybenzolsulfonsäure und Methallylsulfonsäure, als Monomer enthalten. Die (co-)polymeren Polycarboxylate können als Feststoff oder in wässriger Lösung eingesetzt werden. Der Gehalt von Wasch- oder Reinigungsmitteln an (co-)polymeren Polycarboxylaten beträgt vorzugsweise 0,5 Gew.-% bis 20 Gew.-% und insbesondere 3 Gew.-% bis 10 Gew.-%.

Insbesondere bevorzugt sind auch biologisch abbaubare Polymere aus mehr als zwei verschiedenen Monomereinheiten, beispielsweise solche, die als Monomere Salze der Acrylsäure und der Maleinsäure sowie Vinylalkohol bzw. Vinylalkohol-Derivate oder die als Monomere Salze der Acrylsäure und der 2-Alkylallylsulfonsäure sowie Zucker-Derivate enthalten. Weitere bevorzugte Copolymere sind solche, die als Monomere Acrolein und Acrylsäure/Acrylsäuresalze bzw. Acrolein und Vinylacetat aufweisen. Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren und/oder deren Salze.

Eine weitere Substanzklasse mit Buildereigenschaften stellen die Phosphonate dar. Dabei handelt es sich um die Salze von insbesondere Hydroxyalkan- oder Aminoalkanphosphonsäuren. Unter den Hydroxyalkanphosphonsäuren ist die 1-Hydroxyethan-1,1-diphosphonsäure (HEDP) von besonderer Bedeutung. Sie wird insbesondere als Natriumsalz eingesetzt, wobei das Dinatriumsalz neutral und das Tetranatriumsalz alkalisch reagiert. Als Aminoalkanphosphonsäuren kommen insbesondere Ethylendiamintetramethylenphosphonsäure (EDTMP), Diethylentriaminpentamethylenphosphonsäure (DTPMP) sowie deren höhere Homologe in Frage. Sie werden insbesondere in Form der neutral reagierenden Natriumsalze, so zum Beispiel als Hexanatriumsalz der EDTMP oder als Hepta- und Octa-Natriumsalz der DTPMP, eingesetzt. Auch Mischungen aus den genannten Phosphonaten können als organische Builder verwendet werden. Insbesondere die Aminoalkanphosphonate besitzen zudem ein ausgeprägtes Schwermetallbindevermögen.

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen, erhalten werden können. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 g/mol bis 500000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weißdextrine mit höheren Molmassen im Bereich von 2000 g/mol bis 30000 g/mol. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Gewünschtenfalls geeignete Einsatzmengen liegen insbesondere in zeolithhaltigen und/oder silicathaltigen Formulierungen bei 3 Gew.-% bis 15 Gew.-%.

Weitere brauchbare organische Cobuilder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten.

Darüber hinaus können alle Verbindungen, die in der Lage sind, Komplexe mit Erdalkaliionen auszubilden, als Gerüststoffe eingesetzt werden.

Wasch- und Reinigungsmittel können nichtionische, anionische, kationische und/oder amphotere Tenside enthalten.

Als nichtionische Tenside können alle dem Fachmann bekannten nichtionischen Tenside eingesetzt werden. Mit besonderem Vorzug enthalten Wasch- oder Reinigungsmittel nichtionische Tenside aus der Gruppe der alkoxylierten Alkohole. Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 Mol EO pro Mol Alkohol bevorzugt. Zu den bevorzugten ethoxylierten Alkoholen gehören beispielsweise C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt einer ganzen oder einer gebrochenen Zahl entsprechen können. Bevorzugte Alkoholethoxylate weisen eine eingeengte Homologenverteilung auf (narrow range ethoxylates, NRE).

Alternativ oder zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Außerdem können als weitere nichtionische Tenside auch Alkylglykoside der allgemeinen Formel RO(G)ₓ eingesetzt werden, in der R einem primären geradkettigen oder methylverzweigten, insbesondere in 2-Stellung methylverzweigten aliphatischen Rest mit 8 bis 22, vorzugsweise 12 bis 18 C-Atomen entspricht und G das Symbol ist, das für eine Glykoseeinheit mit 5 oder 6 C-Atomen, vorzugsweise für Glucose, steht. Der Oligomerisierungsgrad x, der die Verteilung von Monoglykosiden und Oligoglykosiden angibt, ist eine beliebige Zahl zwischen 1 und 10; vorzugsweise liegt x bei 1,2 bis 1,4.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können eingesetzt werden. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel 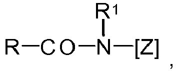 in der R für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgender Acylierung mit einer Fettsäure, einem Fettsäurealkylester oder einem Fettsäurechlorid erhalten werden können. Zur Gruppe der Polyhydroxyfettsäureamide gehören auch Verbindungen der Formel 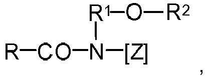 in der R für einen linearen oder verzweigten Alkyl- oder Alkenylrest mit 7 bis 12 Kohlenstoffatomen, R¹ für einen linearen, verzweigten oder zyklischen Alkylrest oder einen Arylrest mit 2 bis 8 Kohlenstoffatomen und R² für einen linearen, verzweigten oder zyklischen Alkylrest oder einen Arylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes. [Z] wird vorzugsweise durch reduktive Aminierung eines reduzierten Zuckers erhalten, beispielsweise Glucose, Fructose, Maltose, Lactose, Galactose, Mannose oder Xylose. Die N-Alkoxy- oder N-Aryloxy-substituierten Verbindungen können durch Umsetzung mit Fettsäuremethylestern in Gegenwart eines Alkoxids als Katalysator in die gewünschten Polyhydroxyfettsäureamide überführt werden.

In Reinigungsmitteln sind Niotenside aus der Gruppe der alkoxylierten Alkohole, besonders bevorzugt aus der Gruppe der gemischt alkoxylierten Alkohole und insbesondere aus der Gruppe der EO/AO/EO-Niotenside, oder der PO/AO/PO-Niotenside, speziell der PO/EO/PO-Niotenside besonders bevorzugt. Solche PO/EO/PO-Niotenside zeichnen sich durch gute Schaumkontrolle aus.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), zum Beispiel die α-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoatkohote und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 Gew.-% bis 5 Gew.-%, eingesetzt.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen, darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen. Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Als weitere anionische Tenside kommen insbesondere Seifen in Betracht. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

An Stelle der genannten Tenside oder in Verbindung mit ihnen können auch kationische und/oder amphotere Tenside eingesetzt werden.

Als kationische Aktivsubstanzen können beispielsweise kationische Verbindungen der nachfolgenden Formeln eingesetzt werden: 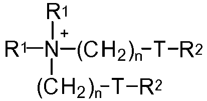 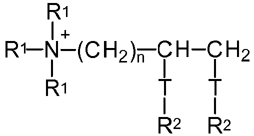 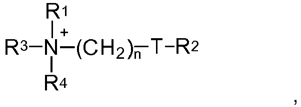 worin jede Gruppe R¹ unabhängig voneinander ausgewählt ist aus C₁₋₆-Alkyl-, -Alkenyl- oder -Hydroxyalkylgruppen; jede Gruppe R² unabhängig voneinander ausgewählt ist aus C₈₋₂₈-Alkyl- oder -Alkenylgruppen; R³ = R¹ oder (CH₂)ₙ-T-R²; R⁴ = R¹ oder R² oder (CH₂)ₙ-T-R²; T = -CH₂-, -O-CO- oder -CO-O- und n eine ganze Zahl von 0 bis 5 ist.

Zur Pflege der Textilien und zur Verbesserung der Textileigenschaften wie einem weicheren "Griff (Avivage) und verringerter elektrostatischer Aufladung (erhöhter Tragekomfort) können textilweichmachende Verbindungen eingesetzt werden. Die Wirkstoffe dieser Formulierungen sind quartäre Ammoniumverbindungen mit zwei hydrophoben Resten, wie beispielsweise das Disteraryldimethylammoniumchlorid, welches jedoch wegen seiner ungenügenden biologischen Abbaubarkeit zunehmend durch quartäre Ammoniumverbindungen ersetzt wird, die in ihren hydrophoben Resten Estergruppen als Sollbruchstellen für den biologischen Abbau enthalten.

Derartige "Esterquats" mit verbesserter biologischer Abbaubarkeit sind beispielsweise dadurch erhältlich, dass man Mischungen von Methyldiethanolamin und/oder Triethanolamin mit Fettsäuren verestert und die Reaktionsprodukte anschließend in an sich bekannter Weise mit Alkylierungsmittein quaterniert. Als Appretur weiterhin geeignet ist Dimethylolethylenharnstoff.

Zur Steigerung der Leistung von Wasch- oder Reinigungsmitteln sind Enzyme einsetzbar. Hierzu gehören insbesondere Proteasen, Amylasen, Lipasen, Hemicellulasen, Cellulasen, Perhydrolasen oder Oxidoreduktasen, sowie vorzugsweise deren Gemische. Diese Enzyme sind im Prinzip natürlichen Ursprungs; ausgehend von den natürlichen Molekülen stehen für den Einsatz in Wasch- und Reinigungsmitteln verbesserte Varianten zur Verfügung, die entsprechend bevorzugt eingesetzt werden. Wasch- oder Reinigungsmittel enthalten Enzyme vorzugsweise in Gesamtmengen von 1 × 10⁻⁶ Gew.-% bis 5 Gew.-% bezogen auf aktives Protein. Die Proteinkonzentration kann mit Hilfe bekannter Methoden, zum Beispiel dem BCA-Verfahren oder dem Biuret-Verfahren bestimmt werden.

Unter den Proteasen sind solche vom Subtilisin-Typ bevorzugt. Beispiele hierfür sind die Subtilisine BPN' und Carlsberg sowie deren weiterentwickelte Formen, die Protease PB92, die Subtilisine 147 und 309, die Alkalische Protease aus Bacillus lentus, Subtilisin DY und die den Subtilasen, nicht mehr jedoch den Subtilisinen im engeren Sinne zuzuordnenden Enzyme Thermitase, Proteinase K und die Proteasen TW3 und TW7.

Beispiele für einsetzbare Amylasen sind die α-Amylasen aus Bacillus licheniformis, aus B. amyloliquefaciens, aus B. stearothermophilus, aus Aspergillus niger und A. oryzae sowie die für den Einsatz in Wasch- und Reinigungsmitteln verbesserten Weiterentwicklungen der vorgenannten Amylasen. Des Weiteren sind für diesen Zweck die α-Amylase aus Bacillus sp. A 7-7 (DSM 12368) und die Cyclodextrin-Glucanotransferase (CGTase) aus B. agaradherens (DSM 9948) hervorzuheben.

Einsetzbar sind wegen ihrer Triglycerid-spaltenden Aktivität Lipasen oder Cutinasen. Hierzu gehören beispielsweise die ursprünglich aus Humicola lanuginosa (Thermomyces lanuginosus) erhältlichen oder aus diesen weiterentwickelten Lipasen, insbesondere solche mit dem Aminosäureaustausch D96L. Des Weiteren sind beispielsweise die Cutinasen einsetzbar, die ursprünglich aus Fusarium solani pisi und Humicola insolens isoliert worden sind. Einsetzbar sind weiterhin Lipasen und/oder Cutinasen, deren Ausgangsenzyme ursprünglich aus Pseudomonas mendocina und Fusarium solanii isoliert worden sind.

Weiterhin können Enzyme eingesetzt werden, die unter dem Begriff Hemicellulasen zusammengefasst werden. Hierzu gehören beispielsweise Mannanasen, Xanthanlyasen, Pektinlyasen (=Pektinasen), Pektinesterasen, Pektatlyasen, Xyloglucanasen (=Xylanasen), Pullulanasen und β-Glucanasen.

Zur Erhöhung der bleichenden Wirkung können gewünschtenfalls Oxidoreduktasen, beispielsweise Oxidasen, Oxygenasen, Katalasen, Peroxidasen, wie Halo-, Chloro-, Bromo-, Lignin-, Glucose- oder Mangan-peroxidasen, Dioxygenasen oder Laccasen (Phenoloxidasen, Polyphenoloxidasen) eingesetzt werden. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluss zu gewährleisten (Mediatoren).

Die Enzyme können in jeder nach dem Stand der Technik etablierten Form eingesetzt werden. Hierzu gehören beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren versetzt. Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil. Weiterhin ist es möglich, zwei oder mehrere Enzyme zusammen zu konfektionieren, so dass ein einzelnes Granulat mehrere Enzymaktivitäten aufweist.

Bevorzugt werden ein oder mehrere Enzyme und/oder Enzymzubereitungen, vorzugsweise Protease-Zubereitungen und/oder Amylase-Zubereitungen, in Mengen von 0,1 Gew.-% bis 5 Gew.-%, vorzugsweise von 0,2 Gew.-% bis 4,5 Gew.-% und insbesondere von 0,4 Gew.-% bis 4 Gew.-%, eingesetzt.

Als Parfümöle oder Duftstoffe können einzelne Riechstoffverbindungen, z.B. synthetische Produkte vom Typ der Ester, Ether, Aldehyde, Ketone, Alkohole und Kohlenwasserstoffe verwendet werden. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, zum Beispiel Pinien-, Citrus-, Jasmin-, Patchouly-, Rosen- oder Ylang-Ylang-Öl. Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 g/mol, während Molmassen von 300 g/mol und darüber eher eine Ausnahme darstellen. Auf Grund der unterschiedlichen Flüchtigkeit von Riechstoffen verändert sich der Geruch eines aus mehreren Riechstoffen zusammengesetzten Parfüms oder Duftstoffs während des Verdampfens, wobei man die Geruchseindrücke in "Kopfnote" (top note), "Herz- oder Mittelnote" (middle note, body) sowie "Basisnote" (end note, dry out) unterteilt. Da die Geruchswahrnehmung zu einem großen Teil auch auf der Geruchsintensität beruht, besteht die Kopfnote eines Parfüms oder Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Bei der nachfolgenden Einteilung der Riechstoffe in "leichter flüchtige" oder "haftfeste" Riechstoffe ist also über den Geruchseindruck und darüber, ob der entsprechende Riechstoff als Kopf- oder Herznote wahrgenommen wird, nichts ausgesagt. Die Duftstoffe können direkt verarbeitet werden, es kann aber auch vorteilhaft sein, die Duftstoffe auf Träger aufzubringen, die durch eine langsamere Duftfreisetzung für langanhaltenden Duft sorgen. Als solche Trägermaterialien haben sich beispielsweise Cyclodextrine bewährt, wobei die Cyclodextrin-Parfüm-Komplexe zusätzlich noch mit weiteren Hilfsstoffen beschichtet werden können.

Bei der Wahl des Färbemittels muss beachtet werden, dass die Färbemittel eine hohe Lagerstabilität und Unempfindlichkeit gegenüber Licht sowie keine zu starke Affinität gegenüber textilen Oberflächen und hier insbesondere gegenüber Kunstfasern aufweisen können. Gleichzeitig ist auch zu berücksichtigen, dass Färbemittel unterschiedliche Stabilitäten gegenüber der Oxidation aufweisen können. Im allgemeinen gilt, dass wasserunlösliche Färbemittel gegen Oxidation stabiler sind als wasserlösliche Färbemittel. Abhängig von der Löslichkeit und damit auch von der Oxidationsempfindlichkeit variiert die Konzentration des Färbemittels in den Wasch- oder Reinigungsmitteln. Bei gut wasserlöslichen Färbemitteln werden typischerweise Färbemittel-Konzentrationen im Bereich von einigen 10⁻² Gew.-% bis 10⁻³ Gew.-% gewählt. Bei den auf Grund ihrer Brillanz insbesondere bevorzugten, allerdings weniger gut wasserlöslichen Pigmentfarbstoffen liegt die geeignete Konzentration des Färbemittels in Wasch- oder Reinigungsmitteln dagegen typischerweise bei einigen 10⁻³ Gew.-% bis 10⁻⁴ Gew.-%. Es werden Färbemittel bevorzugt, die im Waschprozess oxidativ zerstört werden können sowie Mischungen derselben mit geeigneten blauen Farbstoffen, sog. Blautönern. Es hat sich als vorteilhaft erwiesen, Färbemittel einzusetzen, die in Wasser oder bei Raumtemperatur in flüssigen organischen Substanzen löslich sind. Geeignet sind beispielsweise anionische Färbemittel, zum Beispiel anionische Nitrosofarbstoffe.

Zusätzlich zu den bisher genannten Komponenten können die Wasch- oder Reinigungsmittel weitere Inhaltsstoffe enthalten, welche die anwendungstechnischen und/oder ästhetischen Eigenschaften dieser Mittel weiter verbessern. Bevorzugte Mittel enthalten einen oder mehrere Stoffe aus der Gruppe der Elektrolyte, pH-Stellmittel, Fluoreszenzmittel, Hydrotrope, Schauminhibitoren, Silikonöle, Antiredepositionsmittel, optische Aufheller, Vergrauungsinhibitoren, Einlaufverhinderer, Knitterschutzmittel, Farbübertragungsinhibitoren, antimikrobiellen Wirkstoffen, Germizide, Fungizide, Antioxidantien, Antistatika, Bügelhilfsmittel, Phobier- und Imprägniermittel, Quell- und Schiebefestmittel sowie UV-Absorber.

Als Elektrolyte aus der Gruppe der anorganischen Salze kann eine breite Anzahl der verschiedensten Salze eingesetzt werden. Bevorzugte Kationen sind die Alkali- und Erdalkalimetalle, bevorzugte Anionen sind die Halogenide und Sulfate. Aus herstellungstechnischer Sicht ist der Einsatz von NaCl oder MgCl₂ in den Wasch- oder Reinigungsmitteln bevorzugt.

Um den pH-Wert von Wasch- oder Reinigungsmitteln in den gewünschten Bereich zu bringen, kann der Einsatz von pH-Stellmitteln angezeigt sein. Einsetzbar sind hier sämtliche bekannten Säuren oder Laugen, sofern sich ihr Einsatz nicht aus anwendungstechnischen oder ökologischen Gründen oder aus Gründen des Verbraucherschutzes verbietet. Üblicherweise überschreitet die Menge dieser Stellmittel 1 Gew.-% der Gesamtformulierung nicht.

Als Schauminhibitoren, kommen Seifen, Öle, Fette, Paraffine oder Silikonöle in Betracht, die gegebenenfalls auf Trägermaterialien aufgebracht sein können. Als Trägermaterialien eignen sich beispielsweise anorganische Salze wie Carbonate oder Sulfate, Cellulosederivate oder Silikate sowie Mischungen der vorgenannten Materialien. Im Rahmen der vorliegenden Anmeldung bevorzugte Mittel enthalten Paraffine, vorzugsweise unverzweigte Paraffine (n-Paraffine) und/oder Silikone, vorzugsweise linear-polymere Silikone, welche nach dem Schema (R₂SiO)ₓaufgebaut sind und auch als Silikonöle bezeichnet werden. Diese Silikonöle stellen gewöhnlich klare, farblose, neutrale, geruchsfreie, hydrophobe Flüssigkeiten mit einem Molekulargewicht zwischen 1000 g/mol und 150000 g/mol und Viskositäten zwischen 10 mPa·s und 1000000 mPa·s dar.

Als soil repellents kommen die aus dem Stand der Technik bekannten Polymere der Phthalsäure und/oder Terephthalsäure und deren Derivate, insbesondere Polymere aus Ethylenterephthalat und/oder Polyethylenglycolterephthalat oder anionisch und/oder nichtionisch modifizierten Derivaten von diesen, in Frage. Insbesondere bevorzugt von diesen sind die sulfonierten Derivate der Phthalsäure- und Terephthalsäure-Polymere.

Optische Aufheller können insbesondere den Waschmitteln zugesetzt werden, um Vergrauungen und Vergilbungen der behandelten Textilien zu beseitigen. Diese Stoffe ziehen auf die Faser auf und bewirken eine Aufhellung und vorgetäuschte Bleichwirkung, indem sie unsichtbare Ultraviolettstrahlung in sichtbares längerwelliges Licht umwandeln, wobei das aus dem Sonnenlicht absorbierte ultraviolette Licht als schwach bläuliche Fluoreszenz abgestrahlt wird und mit dem Gelbton der vergrauten bzw. vergilbten Wäsche reines Weiß ergibt. Geeignete Verbindungen stammen beispielsweise aus den Substanzklassen der 4,4'-Diamino-2,2'-stilbendisulfonsäuren (Flavonsäuren), 4,4'-Distyryl-biphenylen, Methylumbelliferone, Cumarine, Dihydrochinolinone, 1,3-Diarylpyrazoline, Naphthalsäureimide, Benzoxazol-, Benzisoxazol- und Benzimidazol-Systeme sowie der durch Heterocyclen substituierten Pyrenderivate.

Vergrauungsinhibitoren haben die Aufgabe, den von der Faser abgelösten Schmutz in der Flotte suspendiert zu halten und so das Wiederaufziehen des Schmutzes zu verhindern. Hierzu sind wasserlösliche Kolloide meist organischer Natur geeignet, beispielsweise die wasserlöslichen Salze polymerer Carbonsäuren, Leim, Gelatine, Salze von Ethersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate verwenden, zum Beispiel abgebaute Stärke und Aldehydstärken. Auch Polyvinylpyrrolidon ist brauchbar. Als Vergrauungsinhibitoren einsetzbar sind weiterhin Celluloseether wie Carboxymethylcellulose (Na-Salz), Methylcellulose, Hydroxyalkylcellulose und Mischether wie Methylhydroxyethylcellulose, Methylhydroxypropylcellulose, Methylcarboxy-methylcellulose und deren Gemische. Besonders geeignet sind beispielsweise nichtionische Celluloseether wie Methylcellulose und Methylhydroxypropylcellulose mit einem Anteil an Methoxygruppen von 15 bis 30 Gew.-% und an Hydroxypropylgruppen von 1 bis 15 Gew.-%, jeweils bezogen auf den nichtionischen Celluloseether.

Da textile Flächengebilde, insbesondere aus Reyon, Zellwolle, Baumwolle und deren Mischungen, zum Knittern neigen können, weil die Einzelfasern gegen Durchbiegen, Knicken, Pressen und Quetschen quer zur Faserrichtung empfindlich sind, können synthetische Knitterschutzmittel eingesetzt werden. Hierzu zählen beispielsweise synthetische Produkte auf der Basis von Fettsäuren, Fettsäureestern, Fettsäureamiden, -alkylolestern, -alkylolamiden oder Fettalkoholen, die meist mit Ethylenoxid umgesetzt sind, oder Produkte auf der Basis von Lecithin oder modifizierter Phosphorsäureester.

Phobier- und Imprägnierverfahren dienen der Ausrüstung von Textilien mit Substanzen, welche die Ablagerung von Schmutz verhindern oder dessen Auswaschbarkeit erleichtern. Bevorzugte Phobier- und Imprägniermittel sind perfluorierte Fettsäuren, auch in Form ihrer Aluminium- und Zirkoniumsalze, organische Silikate, Silikone, Polyacrylsäureester mit perfluorierter Alkohol-Komponente oder mit perfluoriertem Acyl- oder Sulfonyl-Rest gekoppelte, polymerisierbare Verbindungen. Auch Antistatika können enthalten sein. Die schmutzabweisende Ausrüstung mit Phobier- und Imprägniermitteln wird oft als eine Pflegeleicht-Ausrüstung eingestuft. Das Eindringen der Imprägniermittel in Form von Lösungen oder Emulsionen der betreffenden Wirkstoffe kann durch Zugabe von Netzmitteln erleichtert werden, welche die Oberflächenspannung herabsetzen. Ein weiteres Einsatzgebiet von Phobier- und Imprägniermitteln ist die wasserabweisende Ausrüstung von Textilwaren, Zelten, Planen, Leder usw., bei der im Gegensatz zum Wasserdichtmachen die Gewebeporen nicht verschlossen werden, der Stoff also atmungsaktiv bleibt (Hydrophobieren). Die zum Hydrophobieren verwendeten Hydrophobiermittel überziehen Textilien, Leder, Papier, Holz usw. mit einer sehr dünnen Schicht hydrophober Gruppen, wie längere Alkyl-Ketten oder Siloxan-Gruppen. Geeignete Hydrophobiermittel sind zum Beispiel Paraffine, Wachse, Metallseifen usw. mit Zusätzen an Aluminium- oder Zirkonium-Salzen, quartäre Ammonium-Verbindungen mit langkettigen Alkyl-Resten, Harnstoff-Derivate, Fettsäure-modifizierte Melaminharze, Chrom-Komplexsalze, Silikone, Zinn-organische Verbindungen und Glutardialdehyd sowie perfluorierte Verbindungen. Die hydrophobierten Materialien fühlen sich nicht fettig an; dennoch perlen - ähnlich wie an gefetteten Stoffen - Wassertropfen an ihnen ab, ohne zu benetzen. So haben zum Beispiel Silikon-imprägnierte Textilien einen weichen Griff und sind wasser- und schmutzabweisend; Flecke aus Tinte, Wein, Fruchtsäften und dergleichen sind leichter zu entfernen.

Zur Bekämpfung von Mikroorganismen können antimikrobielle Wirkstoffe eingesetzt werden. Hierbei unterscheidet man je nach antimikrobiellem Spektrum und Wirkungsmechanismus zwischen Bakteriostatika und Bakteriziden, Fungistatika und Fungiziden und so weiter. Stoffe aus diesen Gruppen sind beispielsweise Benzalkoniumchloride, Alkylarlylsulfonate, Halogenphenole und Phenolmercuriacetat, wobei auch gänzlich auf diese Verbindungen verzichtet werden kann.

Um unerwünschte, durch Luftsauerstoffeinwirkung und andere oxidative Prozesse verursachte Veränderungen an den Waschmitteln und/oder den behandelten Textilien zu verhindern, können die Mittel Antioxidantien enthalten. Zu dieser Verbindungsklasse gehören beispielsweise substituierte Phenole, Hydrochinone, Brenzcatechine und aromatische Amine sowie organische Sulfide, Polysulfide, Dithiocarbamate, Phosphite und Phosphonate.

Ein erhöhter Tragekomfort kann aus der zusätzlichen Verwendung von Antistatika resultieren. Antistatika vergrößern die Oberflächenleitfähigkeit und ermöglichen damit ein verbessertes Abfließen gebildeter Ladungen. Äußere Antistatika sind in der Regel Substanzen mit wenigstens einem hydrophilen Molekülliganden und geben auf den Oberflächen einen mehr oder minder hygroskopischen Film. Diese zumeist grenzflächenaktiven Antistatika lassen sich in stickstoffhaltige (Amine, Amide, quartäre Ammoniumverbindungen), phosphorhaltige (Phosphorsäureester) und schwefelhaltige (Alkylsulfonate, Alkylsulfate) Antistatika unterteilen. Lauryl- (oder Stearyl-) dimethylbenzylammoniumchloride eignen sich ebenfalls als Antistatika für Textilien bzw. als Zusatz zu Waschmitteln, wobei zusätzlich ein Avivageeffekt erzielt wird.

Zur Verbesserung des Wasserabsorptionsvermögens, der Wiederbenetzbarkeit der behandelten Textilien und zur Erleichterung des Bügelns der behandelten Textilien können in Textilwaschmitteln Silikonderivate eingesetzt werden. Diese verbessern zusätzlich das Ausspülverhalten von Wasch- oder Reinigungsmitteln durch ihre schauminhibierenden Eigenschaften. Bevorzugte Silikonderivate sind beispielsweise Polydialkyl- oder Alkylarylsiloxane, bei denen die Alkylgruppen ein bis fünf C-Atome aufweisen und ganz oder teilweise fluoriert sind. Bevorzugte Silikone sind Polydimethylsiloxane, die gegebenenfalls derivatisiert sein können und dann aminofunktionell oder quaterniert sind oder Si-OH-, Si-H- und/oder Si-Cl-Bindungen aufweisen. Weitere bevorzugte Silikone sind die Polyalkylenoxid-modifizierten Polysiloxane, also Polysiloxane, welche beispielsweise Polyethylenglykole aufweisen, sowie die Polyalkylenoxid-modifizierten Dimethylpolysiloxane.

Schließlich können auch UV-Absorber eingesetzt werden, die auf die behandelten Textilien aufziehen und die Lichtbeständigkeit der Fasern verbessern. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung phenylsubstituierte Acrylate (Zimtsäurederivate), gegebenenfalls mit Cyanogruppen in 2-Stellung, Salicylate, organische Ni-Komplexe sowie Naturstoffe wie Umbelliferon und die körpereigene Urocansäure geeignet.

Proteinhydrolysate sind auf Grund ihrer faserpflegenden Wirkung weitere geeignete Aktivsubstanzen. Proteinhydrolysate sind Produktgemische, die durch sauer, basisch oder enzymatisch katalysierten Abbau von Proteinen (Eiweißen) erhalten werden. Proteinhydrolysate sowohl pflanzlichen als auch tierischen Ursprungs können eingesetzt werden. Tierische Proteinhydrolysate sind beispielsweise Elastin-, Kollagen-, Keratin-, Seiden- und Milcheiweiß-Proteinhydrolysate, die auch in Form von Salzen vorliegen können. Bevorzugt ist die Verwendung von Proteinhydrolysaten pflanzlichen Ursprungs, zum Beispiel Soja-, Mandel-, Reis-, Erbsen-, Kartoffel- und Weizenproteinhydrolysate. Wenngleich der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin oder Pyroglutaminsäure eingesetzt werden. Ebenfalls möglich ist der Einsatz von Derivaten der Proteinhydrolysate, beispielsweise in Form ihrer Fettsäure-Kondensationsprodukte.

### Beispiele

### Beispiel 1: Synthese erfindungsgemäßer Verbindungen

### 1. Synthese und Analytik von 3-((^{tert}Butyldiphenylsilyl)oxy)-2-methyl-4H-pyran-4-on (3c)

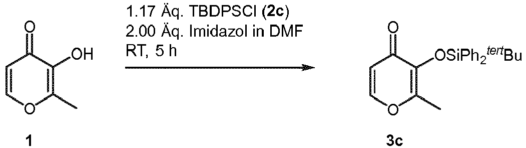

In einem sekurierten Schlenkkolben wurden 2,52 g (20,0 mmol) Maltol (1) und 6,42 g (23,4 mmol) tert-Butyldiphenylsilylchlorid TBDPSCI (2c) vorgelegt und in 50 ml trockenem N,N-Dimethylformamid gelöst. Es wurde 10 min bei Raumtemperatur (20 °C), bis zum vollständigen Auflösen, gerührt. Nach dieser Zeit wurden in einer Portion 2,73 g (40,0 mmol) Imidazol zugegeben und dann 5 h bei 20 °C gerührt. Nach verstrichener Reaktionszeit wurde der Reaktionsmischung solange gesättigte Natriumbicarbonat-Lösung zugesetzt, bis keine Gasentwicklung mehr zu beobachten war. Der entstandene Feststoff wurde durch Zugabe von deionisierten Wasser gelöst und die wässrige Phase anschließend viermal mit jeweils 250 ml eines n-Hexan-Ethylacetat-Gemisches (1:1, v/v) extrahiert. Die organischen Phasen wurden vereinigt und mit wasserfreiem Natriumsulfat getrocknet. Abschließend wurde das erhaltene Rohprodukt säulenchromatographisch an Kieselgel mit einem Lösungsmittelgemisch aus n-Hexan und Ethylacetat (3:1, v/v) gereinigt. Man erhielt 7,03 g (19,3 mmol, 96 %) des Produkts 3c in Form eines farblosen Feststoffes.

Schmelzpunkt: 127°C.

Rf-Wert (n-Hexan:EtOAc, 5:1): 0,22.

¹H-NMR (300 MHz, Chloroform-d): δ = 1,08 (s, 9 H), 2,37 (s, 3 H), 6,09 (d, J = 5,6 Hz, 1 H), 7,30-7,42 (m, 6 H), 7,48 (d, J = 5,6 Hz, 1 H), 7,69-7,73 (m, 4 H).

### 2. a) Allgemeine Arbeitsvorschrift zur Aminierung des geschützten Maltolvorläufers 3c

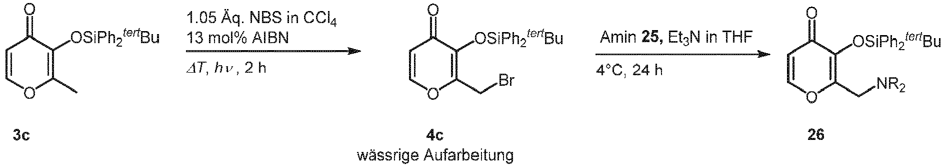

In einem sekurierten Schlenkrohr wurde das Pyron 3c vorgelegt und in 5 ml Tetrachlormethan gelöst. Anschließend wurden 187 mg (1,05 mmol) N-Bromsuccinimid zugegeben und das Reaktionsgemisch wurde auf 96 °C erhitzt. Nach 15 min wurden 21,9 mg (13 mol%) Azobis(isobutyronitril) im Stickstoffgegenstrom zugegeben und die Reaktionsmischung 2 h lang unter Bestrahlung mit einer UV-Lampe im Ölbad erhitzt. Anschließend wurde die Reaktionsmischung abgekühlt und in Dichlormethan aufgenommen. Die Lösung wurde zweimal mit gesättigter wässriger Natriumhydrogencarbonatlösung gewaschen. Die vereinigten wässrigen Phasen wurden dreimal mit jeweils 10 ml Dichlormethan extrahiert. Die vereinigten organischen Phasen wurden mit gesättigter wässriger Natiumchloridlösung gewaschen und die wässrige Phase wurde zweimal mit jeweils 10 ml Dichlormethan extrahiert. Die vereinigten organischen Phasen wurden mit wasserfreiem Na₂SO₄ getrocknet und das Lösungsmittel wurde vollständig abdestilliert. Das erhaltene Rohprodukt wurde in 4 ml Tetrahydrofuran gelöst. In einem sekurierten 50 ml-Zweihalskolben wurde 5 ml Tetrahydrofuran vorgelegt und das Amin 25 zugegeben. Der Kolben wurde im Kühlschrank auf 4 °C abgekühlt, dann wurde Triethylamin zugegeben, umgeschwenkt und wieder im Kühlschrank für mindestens 30 min gekühlt. Nach dieser Zeit wurde das in Tetrahydrofuran gelöste Rohprodukt zügig in drei Portionen zugegeben und der Reaktionsansatz 24 h im Kühlschrank gelagert. Danach wurde auf Raumtemperatur erwärmt und wie oben beschrieben aufgearbeitet. Das erhaltene Rohprodukt wurde an Celite^{®} adsorbiert und säulenchromatographisch an Kieselgel gereinigt.

| Pyron 3c mg (mmol) | Amin 25 mg (mmol) | Triethylamin mg (mmol) | Produkt 26 Ausbeute in mg (%) | |
|---|---|---|---|---|
| 365(1) | 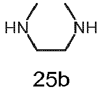 | 134(1,32) | 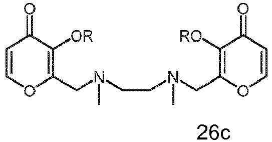 | R = -SiPh₂*^{tert}*Bu |
| | 41.2 (0.47) | | 237 (62) | |
| 365(1) | 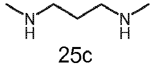 | 135(1,33) | 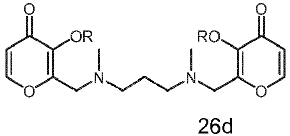 | R = -SiPh₂*^{tert}*Bu |
| | 48.4 (0.46) | | 204 (54) | |
| 365(1) | 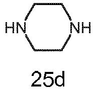 | 135(1,33) | 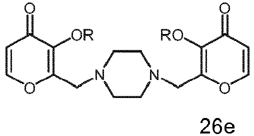 | R = -SiPh₂*^{tert}*Bu |
| | 39.7 (0.46) | | 169 (46) | |

### 2.b) Herstellung von 2,2'-((Ethan-1,2-diyl-bis(methylazandiyl))bis(methylen))bis(3-((tert-butyldiphe-nylsilyl)oxy)-4H-pyran-4-on) (26c)

Analog zur allgemeinen Arbeitsvorschrift (2.a) wurden nach einer säulenchromatographischen Reinigung an Kieselgel mit einem Gemisch bestehend aus n-Hexan und Ethylacetat (1:1, v/v) und einem Methanolgradienten (0 % nach 10 % v/v) als Eluenten 237 mg (0.29 mmol, 62 %) Produkt 26c als leicht orangener Feststoff analysenrein gewonnen werden.

Schmelzpunkt: 194 °C.

Rf-Wert (CH₂Cl₂:MeOH, 10:1): 0.47.

¹H-NMR (300 MHz, Chloroform-d): δ = 1,05 (s, 18 H), 2,40 (s, 6 H), 2,73 (s (breit), 4 H), 3,80 (s (breit), 4 H), 6,09 (d, J = 5,6 Hz, 2 H), 7,27-7,41 (m, 12 H), 7,55 (d, J = 5,6 Hz, 2 H), 7,61-7,75 (m, 8 H).

### 2.c) Herstellung von 2,2'-((Propan-1,3-diylbis(methylazandiyl))bis(methylen))bis(3-((tert-butyldiphe-nylsilyl)oxy)-4H-pyran-4-on) (26d)

Analog zur allgemeinen Arbeitsvorschrift (2.a) wurden nach einer säulenchromatographischen Reinigung an Kieselgel mit einem Gemisch bestehend aus n-Hexan und Ethylacetat (1:1, v/v) und einem Methanolgradienten (0 % nach 10 % v/v) als Eluenten 204 mg (0,25 mmol, 54 %) Produkt 26d als leicht orangener Feststoff analysenrein gewonnen.

Schmelzpunkt: 145 °C.

Rf-Wert (CH₂Cl₂:MeOH, 10:1): 0,36.

¹H-NMR (300 MHz, Chloroform-d): δ = 1,05 (s, 18 H), 1,71-1,86 (m, 2 H), 2,37 (s, 6 H), 2,55 (t, J = 7,2 Hz, 4 H), 3,72 (s, 4 H), 6,07 (d, J = 5,6 Hz, 2 H), 7,28-7,41 (m, 12 H), 7,53 (d, J = 5,6 Hz, 2 H), 7,67-7,70 (m, 8 H).

### 2.d) Herstellung von von 2,2'-(Piperazin-1,4-diylbis(methylen))bis(3-((tert-butyldiphenyl-silyl)oxy)-4H-pyran-4-on) (26e)

Analog zur allgemeinen Arbeitsvorschrift (2.a) wurden nach einer säulenchromatographischen Reinigung an Kieselgel mit einem Gemisch bestehend aus n-Hexan und Ethylacetat (1:1, v/v) und einem Methanolgradienten (0 % nach 10 % v/v) als Eluenten 169 mg (0,21 mmol, 46 %) des Produkts 26e als leicht beiger Feststoff analysenrein gewonnen.

Schmelzpunkt: 240 °C.

¹H-NMR (300 MHz, Chloroform-d): δ = 1,05 (s, 18 H), 2,69 (s (breit), 8 H), 3,76 (s, 4 H), 6,10 (d, J = 5,6 Hz, 2 H), 7,27-7,42 (m, 12 H), 7,60 (d, J = 5,6 Hz, 2 H), 7,64-7,73 (m, 8 H).

### 3. Herstellung von 2,2'-((Ethan-1,2-diylbis(methylazanediyl))bis(methylen))bis(3-hydroxy-4H-pyran-4-on)-Hydrochlorid (28b)

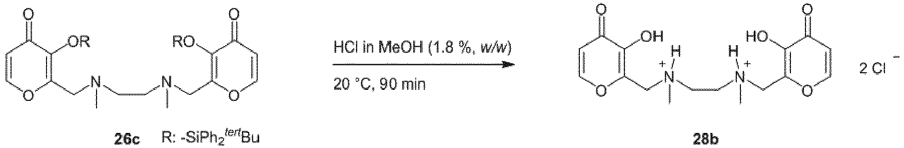

5 g konzentrierte Salzsäure wurden langsam in 95 g Methanol eingetragen. In einem sekurierten Schlenkrohr wurden 407 mg (0,50 mmol) des Maltoderivats 26c vorgelegt und das Gefäß nochmals evakuiert und mit Stickstoff belüftet. Anschließend wurden unter Rühren im Stickstoffgegenstrom 2,5 ml der HCl-Lösung zugegeben. Nach 15 min trübte sich die gelbe klare Reaktionslösung und es fiel ein farbloser Feststoff aus. Nach 90 min wurde die Reaktionsmischung aufgearbeitet. Der entstandene Feststoff wurde dreimal mit Methanol gewaschen. Nach der Trocknung am Hochvakuum erhielt man 152 mg (0,37 mmol, 74 %) 28b in Form eines farblosen Feststoffs. Darüber hinaus konnten weitere 42 mg (0,10 mmol, 20 %) des Produktes 28b aus der Mutterlauge nach Abdestillieren des Lösungsmittels und dreimaligem Resuspendieren in Dichlormethan gewonnen werden. Das entspricht einer Gesamtausbeute von 194 mg (0.47 mmol, 94 %).

¹H-NMR (300 MHz, Deuteriumoxid-d₂): δ = 3,.03 (s, 6 H), 3,79 (s(breit), 4 H), 4,59 (s (breit), 4 H), 6,62 (d, J = 5,6 Hz, 2 H), 8,13 (d, J = 5,6 Hz, 2 H).

¹³C-NMR (75 MHz, D₂O): δ = 41,7 (2 CH₃), 50,1 (2 CH₂), 52,9 (2 CH₂), 114,8 (2 CH), 141,3 (2 Cquart), 147,6 (2 Cquart), 157,9 (2 CH), 176,1 (2 Cquart).

Analog wurden 26d und 26e zu den Verbindungen 28c und 28d entschützt.

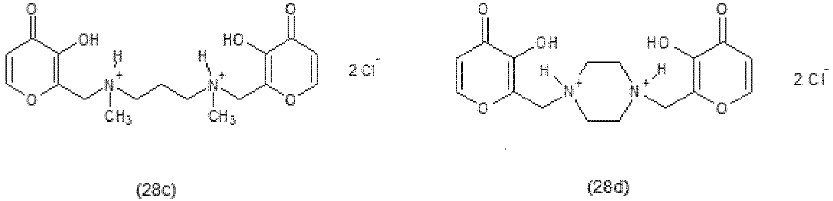

### Beispiel 3: Reinigungsleistung

Es wurden Waschversuche bei 40 °C an verschiedenen in der nachfolgenden Tabelle 1 angegebenen Anschmutzungen, die auf polyphenolischen natürlichen Farbstoffen (Flavonoiden) beruhen, durchgeführt. Die Herstellung der Testanschmutzungen erfolgte manuell durch Dosieren einer konstanten Menge einer verdünnten wässrigen Lösung der angegebenen Extrakte auf Baumwollgewebe und anschließendes Trocknen. Für die Waschversuche wurde ein bleichmittelfreies Flüssigwaschmittel (FWM) verwendet und damit Waschlaugen mit pH 8,5 angesetzt, bestehend aus 69 g FWM und gegebenenfalls 1,4 g eines der ebenfalls in Tabelle 1 angegebenen Wirkstoffe auf 17 I Wasser von 16 °dH. Die Auswertung erfolgte über Farbabstandsmessung gemäß der L*a*b* - Werte und der daraus berechneten Y-Werte als Maß für die Helligkeit. Die folgende Tabelle zeigt die Verbesserung der Fleckentfernung als Unterschied (Differenz Y(nach dem Waschen) - Y(vor dem Waschen)) zwischen der FWM-Rezeptur mit zugesetztem Wirkstoff und dem FWM ohne zugesetztem Wirkstoff als Mittelwerte von Dreifachbestimmung.

**Tabelle 1: Helligkeitsdifferenzunterschiede**

| Anschmutzung mit Extrakt aus | FWM + 28b | FWM + 28c | FWM + 28d |
|---|---|---|---|
| Blaubeere | 6,3 | 4,7 | 5,5 |
| Heidelbeere | 5,4 | 3,7 | 3,7 |

Die dY-Werte bei Zusatz eines erfindungsgemäß verwendeten Wirkstoffs waren immer größer als die mit dem FWM ohne Zusatz erzielten Werte, was einem höheren Weißgrad und somit einer verbesserten Fleckentfernung entspricht.

## Patentansprüche

1. Verwendung von Verbindungen der allgemeinen Formel (I), 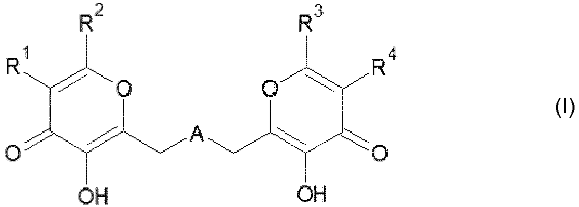 in der A für -Q-(CH₂)ₙ-Q, -(CH₂)ₘ-Q-(CH₂)ₚ- oder 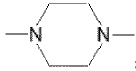 steht, Q für O, N(R⁵) oder N⁺H(R⁵) X⁻ steht, R¹, R², R³, R⁴ und R⁵ unabhängig voneinander für Wasserstoff oder eine geradkettige oder verzweigte Alkylgruppe mit 1 bis 4 C-Atomen stehen, X⁻ für ein ladungsausgleichendes Anion steht, und n für eine Zahl von 1 bis 10 steht, und m und p unabhängig voneinander für Zahlen von 0 bis 7 stehen,
in Wasch- oder Reinigungsmitteln zur Verbesserung der Wasch- oder Reinigungsleistung gegenüber bleichbaren Anschmutzungen.

2. Verwendung nach Anspruch 1, **dadurch gekennzeichnet, dass** die Anschmutzungen polymerisierbare Substanzen, ausgewählt aus polyphenolischen Farbstoffen, insbesondere aus Flavonoiden, vor allem aus Farbstoffen der Klasse der Anthocyanidine oder Anthocyane oder Oligomeren dieser Verbindungen, enthält.

3. Verwendung nach Anspruch 1 oder 2, **dadurch gekennzeichnet, dass** die verbesserte Wasch- oder Reinigungsleistung in einer verbesserten Entfernung von grün-, gelb-, rot-, blau-, violett, lila-, braun-, purpur- oder rosafarbigen Anschmutzungen, insbesondere von Flecken von Gras, Früchten oder Gemüse, insbesondere von Anschmutzungen durch Lebensmittelprodukte, wie Gewürze, Saucen, Chutneys, Currys, Pürees und Marmeladen, oder Getränke, wie Kaffee, Tee, Weine und Säfte, die entsprechende grüne, gelbe, rote, violette, lilafarbene, braune, purpurfarbene, rosafarbene und/oder blaue Farbstoffe enthalten, besteht.

4. Verwendung nach einem der Ansprüche 1 bis 3, **dadurch gekennzeichnet, dass** die Anschmutzungen ausgewählt sind aus Anschmutzungen durch Kirsche, Morelle, Traube, Apfel, Granatapfel, Aronia, Pflaume, Sanddorn, Açai, Kiwi, Mango, Gras, oder Beeren, insbesondere durch rote oder schwarze Johannisbeeren, Holunderbeeren, Brombeeren, Himbeeren, Blaubeeren, Preiselbeeren, Kronsbeeren, Erdbeeren oder Heidelbeeren, durch Kaffee, Tee, Rotkohl, Blutorange, Aubergine, Tomate, Karotte, Rote Beete, Spinat, Paprika, rotfleischige oder blaufleischige Kartoffel, oder rote Zwiebel.

5. Wasch- oder Reinigungsmittel, enthaltend eine Verbindung der allgemeinen Formel (I), 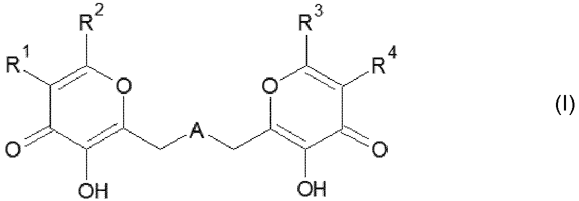 in der A für -Q-(CH₂)ₙ-Q, -(CH₂)ₘ-Q-(CH₂)ₚ- oder 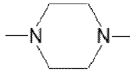 steht, Q für O, N(R⁵) oder N⁺H(R⁵) X⁻ steht, R¹, R², R³, R⁴ und R⁵ unabhängig voneinander für Wasserstoff oder eine geradkettige oder verzweigte Alkylgruppe mit 1 bis 4 C-Atomen stehen, X⁻ für ein ladungsausgleichendes Anion steht, und n für eine Zahl von 1 bis 10 steht, und m und p unabhängig voneinander für Zahlen von 0 bis 7 stehen.

6. Mittel nach Anspruch 5, **dadurch gekennzeichnet, dass** es 0,001 Gew.-% bis 20 Gew.-%, insbesondere 0,01 Gew.-% bis 10 Gew.-% einer Verbindung der allgemeinen Formel (I) enthält.

7. Mittel nach Anspruch 5 oder 6, **dadurch gekennzeichnet, dass** es keine oxidativen Bleichmittel im engeren Sinne, also Hypochlorite, Wasserstoffperoxid oder Wasserstoffperoxid liefernde Substanzen und Peroxosäuren, enthält.

8. Mittel nach einem der Ansprüche 5 bis 7, **dadurch gekennzeichnet, dass** es neben der Verbindung der allgemeinen Formel (I) weitere Bestandteile von Textilwaschmitteln, ausgewählt aus der Gruppe der Gerüststoffe und Tenside, enthält.

9. Verwendung nach einem der Ansprüche 1 bis 4 oder Mittel nach einem der Ansprüche 5 bis 8, **dadurch gekennzeichnet, dass** in den Verbindungen der allgemeinen Formel R¹ und R³ und/oder R² und R⁴ gleich sind.

10. Verwendung oder Mittel nach einem der vorhergehenden Ansprüche, **dadurch gekennzeichnet, dass** in den Verbindungen der allgemeinen Formel R¹, R², R³ und R⁴ Wasserstoff sind, und/oder R⁵ eine Methylgruppe ist, und/oder n eine Zahl von 1 bis 6, insbesondere 1 bis 3 ist.

## Claims

1. The use of compounds of general formula (I), 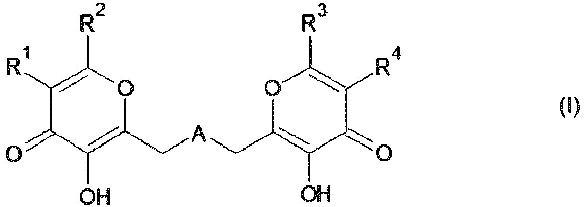 in which A represents -Q-(CH₂)ₙ-Q, -(CH₂)ₘ-Q-(CH₂)ₚ- or 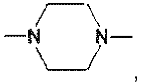 , Q represents O, N(R⁵) or N⁺H(R⁵) X⁻, R¹, R², R³, R⁴ and R⁵ represent, independently of one another, hydrogen or a straight-chain or branched alkyl group having 1 to 4 C atoms, X⁻ represents a charge-balancing anion, and n represents a number from 1 to 10, and m and p represent, independently of one another, numbers from 0 to 7,
in washing or cleaning agents for improving the washing or cleaning performance against bleachable stains.

2. The use according to claim 1, **characterized in that** the stains contain polymerizable substances, selected from polyphenolic dyes, in particular flavonoids, more particularly dyes from the class of anthocyanidins or anthocyanins or oligomers of said compounds.

3. The use according to claim 1 or 2, **characterized in that** the improved washing or cleaning performance consists of improved removal of green, yellow, red, blue, violet, lilac, brown, purple or pink stains, in particular marks caused by grass, fruits or vegetables, in particular stains caused by food products, such as spices, sauces, chutneys, curries, purees and jams, or beverages, such as coffee, tea, wines and juices, which contain corresponding green, yellow, red, violet, lilac, brown, purple, pink, and/or blue dyes.

4. The use according to one of claims 1 to 3, **characterized in that** the stains are selected from stains caused by cherries, morellos, grapes, apples, pomegranates, chokeberries, plums, sea buckthorns, açai berries, kiwis, mangoes, grass or berries, in particular caused by redcurrants, blackcurrants, elderberries, blackberries, raspberries, blueberries, lingonberries, cranberries, strawberries or bilberries, or by coffee, tea, red cabbage, blood orange, eggplant, tomato, carrot, beetroot, spinach, bell pepper, red or blue potato or red onion.

5. A washing or cleaning agent containing a compound of general formula (I), 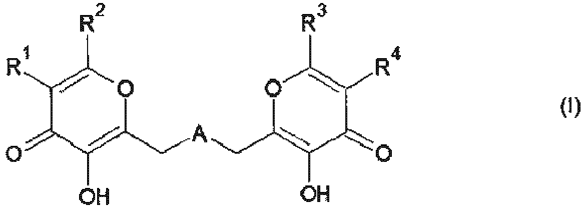 in which A represents -Q-(CH₂)ₙ-Q, -(CH₂)ₘ-Q-(CH₂)ₚ- or 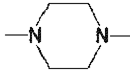 , Q represents O, N(R⁵) or N⁺H(R⁵) X⁻, R¹, R², R³, R⁴ and R⁵ represent, independently of one another, hydrogen or a straight-chain or branched alkyl group having 1 to 4 C atoms, X⁻ represents a charge-balancing anion, and n represents a number from 1 to 10, and m and p represent, independently of one another, numbers from 0 to 7.

6. The agent according to claim 5, **characterized in that** it contains 0.001 wt.% to 20 wt.%, in particular 0.01 wt.% to 10 wt.%, of a compound of general formula (I).

7. The agent according to claim 5 or 6, **characterized in that** it does not contain oxidative bleaching agents in the narrower sense, i.e. hypochlorites, hydrogen peroxide or substances that yield hydrogen peroxide, and peroxoacids.

8. The agent according to one of claims 5 to 7, **characterized in that** it contains, in addition to the compound of general formula (I), further constituents of textile-washing agents, selected from the group of builders and surfactants.

9. The use according to one of claims 1 to 4 or the agent according to one of claims 5 to 8, **characterized in that**, in the compounds of the general formula, R¹ and R³ and/or R² and R⁴ are the same.

10. The use or the agent according to one of the preceding claims, **characterized in that**, in the compounds of the general formula, R¹, R², R³ and R⁴ are hydrogen, and/or R⁵ is a methyl group, and/or n is a number from 1 to 6, in particular 1 to 3.

## Revendications

1. Utilisation de composés de formule générale (I), 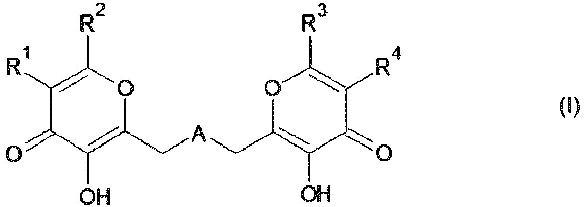 dans laquelle A représente -Q-(CH₂)ₙ-Q, -(CH₂)ₘ-Q-(CH₂)_{P}- ou 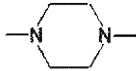 , Q représente O, N(R⁵) ou N⁺H(R⁵)X⁻, R¹, R², R³, R⁴ et R⁵ représentent, indépendamment les uns des autres, de l'hydrogène ou un groupe alkyle linéaire ou ramifié comportant 1 à 4 atomes de carbone, X⁻ représente un anion d'équilibrage de charge, et n représente un nombre de 1 à 10, et m et p représentent, indépendamment l'un de l'autre, des nombres de 0 à 7,
dans des agents de lavage ou des agents de nettoyage permettant d'améliorer la performance de lavage ou de nettoyage contre les salissures blanchissables.

2. Utilisation selon la revendication 1, **caractérisée en ce que** les salissures contiennent des substances polymérisables choisies parmi des colorants polyphénoliques, en particulier parmi des flavonoïdes, notamment parmi des colorants de la classe des anthocyanidines ou des anthocyanes ou des oligomères desdits composés.

3. Utilisation selon la revendication 1 ou 2, **caractérisée en ce que** la performance de lavage ou de nettoyage améliorée consiste en une élimination améliorée de salissures vertes, jaunes, rouges, bleues, violettes, mauves, brunes, pourpres ou roses, en particulier de taches de gazon, de fruits ou de légumes, en particulier de salissures causées par des produits alimentaires, tels que les épices, les sauces, les chutneys, les currys, les purées et les confitures, ou par des boissons, telles que le café, le thé, les vins et les jus contenant des colorants verts, jaunes, rouges, violets, mauves, bruns, pourpres, roses et/ou bleus correspondants.

4. Utilisation selon l'une des revendications 1 à 3, **caractérisée en ce que** les salissures sont choisies parmi les salissures causées par la cerise, la morille, le raisin, la pomme, la grenade, l'aronia, la prune, l'argousier, l'açaï, le kiwi, la mangue, l'herbe ou les baies, en particulier causées par les groseilles ou les cassis, les baies de sureau, les mûres, les framboises, les bleuets, les airelles, les canneberges, les fraises ou les myrtilles, par le café, le thé, le chou rouge, l'orange sanguine, l'aubergine, la tomate, la carotte, la betterave rouge, les épinards, les poivrons, les pommes de terre à chair rouge ou bleue ou l'oignon rouge.

5. Agent de lavage ou agent de nettoyage contenant un composé de formule générale (I), 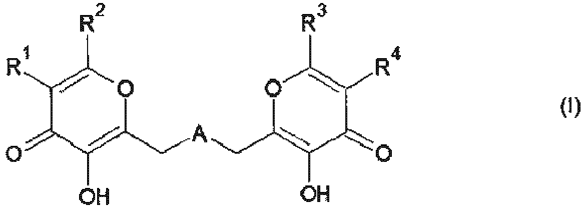 dans laquelle A représente -Q-(CH₂)ₙ-Q, -(CH₂)ₘ-Q-(CH₂)ₚ- ou 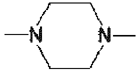 ,Q représente O, N(R⁵) ou N⁺H(R⁵)X⁻, R¹, R², R³, R⁴ et R⁵ représentent, indépendamment les uns des autres, de l'hydrogène ou un groupe alkyle linéaire ou ramifié comportant 1 à 4 atomes de carbone, X⁻ représente un anion d'équilibrage de charge, et n représente un nombre de 1 à 10, et m et p représentent, indépendamment l'un de l'autre, des nombres de 0 à 7.

6. Agent selon la revendication 5, **caractérisé en ce qu'**il contient 0,001 % en poids à 20 % en poids, en particulier 0,01 % en poids à 10 % en poids, d'un composé de formule générale (I).

7. Agent selon la revendication 5 ou 6, **caractérisé en ce qu'**il ne contient aucun agent de blanchiment oxydatif au sens strict, c'est-à-dire des hypochlorites, du peroxyde d'hydrogène ou des substances générant du peroxyde d'hydrogène et des peroxoacides.

8. Agent selon l'une des revendications 5 à 7, **caractérisé en ce qu'**il contient, outre le composé de formule générale (I), d'autres composants d'agents de lavage pour textile choisis dans le groupe des adjuvants et des tensioactifs.

9. Utilisation selon l'une des revendications 1 à 4 ou agent selon l'une des revendications 5 à 8, caractérisé(e) en ce que R¹ et R³ et/ou R² et R⁴ sont identiques dans les composés de formule générale.

10. Utilisation ou agent selon l'une des revendications précédentes, caractérisé(e) en ce que R¹, R², R³ et R⁴ représentent l'hydrogène, et/ou R⁵ représente un groupe méthyle, et/ou n représente un nombre de 1 à 6, en particulier de 1 à 3, dans les composés de formule générale.
